# EUROPEAN PATENT APPLICATION

(11) **EP 1 484 018 A1**
(43) Date of publication of application: **08.12.2004**
(21) Application number: 04012974.4
(22) Date of filing: 02.06.2004
(51) Int. Cl.: A61B 7/04

(54) **Hydraulic amplifier and detector device comprising this amplifier**

(30) Priority: 04.06.2003 IT TO20030416
(71) Applicant: TECNODI S.r.L., 20123 Milano (IT)
(72) Inventor: Menegatti, Barbara Antonella, 20016 Pero (MI) (IT); Pietra, Ugo, 20131 Milano (IT); Pietra, Carlo, 20132 Milano (IT)
(74) Representative: Deambrogi, Edgardo

(57) **Abstract**

A device for amplifying elastic waves or mechanical oscillations in general and converting them into electrical signals is described, comprising:
a rigid cavity forming a chamber (40) for collecting an elastic wave, or for intercepting a mechanical oscillation, this chamber being suitable for application to a body or a machine to be examined, and being arranged to collect, by means of an oscillating moving member (50) positioned in a detecting section, the elastic waves or mechanical oscillations generated by members of the body or machine, and
a transducer device (52) housed inside the aforesaid chamber (40), including a moving element for converting the elastic wave or intercepted mechanical oscillation into an electrical or electromagnetic signal for subsequent processing,
in which the chamber (40) is shaped to house the transducer device (52) in such a way that it is exposed to a field of elastic waves inside the chamber (40) only through the aforesaid moving element,
in which the moving element, has a surface exposed to the field of elastic waves which has a smaller area than the area of the detection section of the cavity, the excursion of the said element being increased, with respect to the excursion of the oscillating element (50) positioned in the detection section, according to the ratio between the area of the detection surface and the area of the moving element exposed to the field of elastic waves.

## Description

The present invention relates in general to an amplifier device for the conversion of small mechanical movements, for example elastic vibrations and mechanical oscillations in general, to proportional electrical signals, which uses the principle of the hydraulic press as described by Pascal, and a detector device comprising this amplifier, provided with an integrated microphone capsule, for generating an amplified electrical signal suitable for subsequent processing.

Instruments for clinical auscultation, for the auscultation of heartbeats and body sounds, are known in the prior art, particularly in the field of medical devices.

The stethoscope is an instrument consisting of a conical acoustic tube whose base of smaller diameter is applied to the ear while the other base is applied to the patient. The stethoscope has the characteristic of permitting the auscultation of restricted areas, but its amplification power is very limited.

A phonendoscope is an instrument consisting of a funnel-shaped cup closed by an elastic membrane in the part having the larger diameter. The smaller diameter is connected to one or two tubes which carry the sound to the ears. The phonendoscope is an instrument which is not very suitable for diagnosis, due to the resonances which modify and attenuate certain sound frequencies.

There are other instruments for electronic auscultation which use various electrodynamic principles for processing the detected frequencies. These instruments are not widely used because of the considerable variations which they cause indirectly in the quality of the reproduced sound.

The use of existing auscultation instruments is certainly favoured by the large and exhaustive body of literature, which can provide an extremely wide range of information, and the habit of using these instruments.

The resolution capacity of the human ear is greater than that of many instruments available in a clinical laboratory; however, the development of instruments for acoustic assistance is significantly impeded by the differences in the amount of energy required to enable the human ear to hear sounds at different frequencies.

The amount of energy required in some cases becomes incompatible with the practicality and portability of the instruments.

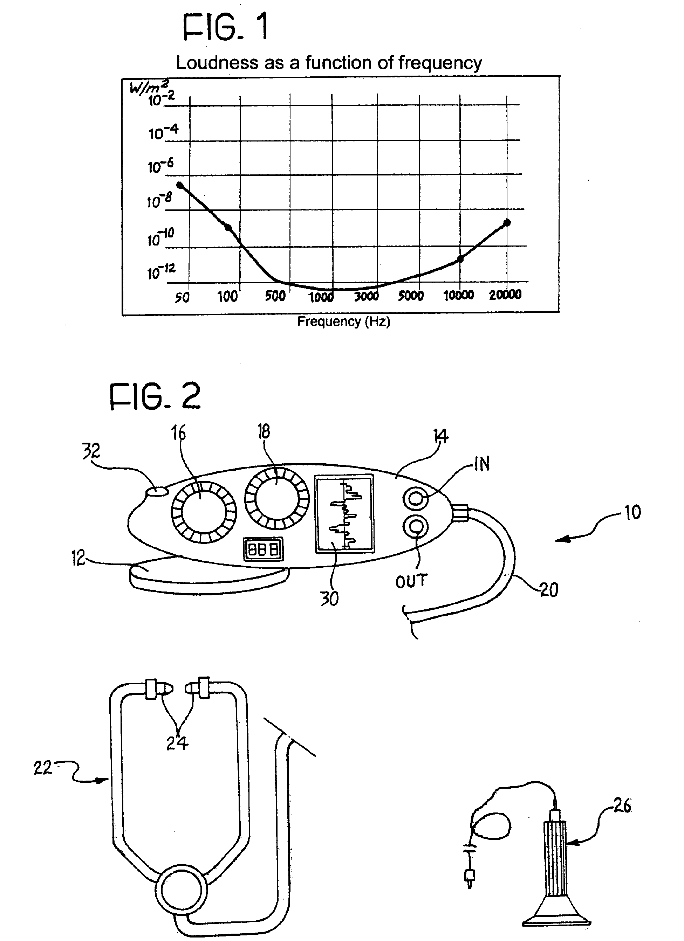

The graph in Figure 1 shows the curve of loudness. For example, only 10⁻¹³ W/m² is required to enable the human ear to perceive a sound at 6 kHz, while a power of 10⁻⁷ W/m², in other words a million times greater, is required for the detection of a sound at 80 Hz.

This makes it necessary to move large bodies of air, as in the case where bulky loudspeakers are used for bass tones.

The conventional acoustic phonendoscope amplifies and makes audible only those sounds which resonate with the shape and volume of the harmonic chamber of its cup, while other sounds are attenuated. In some more advanced phonendoscopes, the amplified frequency can be modified by pressing the capsule with a finger.

The conventional phonendoscope is an unsuitable instrument for diagnosis, because of the low auditory volume, the sound distortion which it produces, and the limited frequency range reproduced. A further deficiency is the impossibility of certifying diagnosis, since this instrument cannot produce documents or graphs to be used as evidence or for wider consultation.

Electronic stethoscopes based on frequency synthesis are in use at the present time. In these, the frequencies of the sounds produced by the human body are multiplied or divided to bring them into the audible band. However, the change in frequency also changes the sounds, which no longer resemble the original sounds and contain annoying background noise.

The object of the invention is to provide an amplifier-transducer device for converting small mechanical movements, for example elastic vibrations and mechanical oscillations in general, into amplified electrical signals, and a detector device, such as an auscultation device, which represents an advance on conventional stethoscopes and phonendoscopes, overcoming the drawbacks of the known art without adversely affecting the practicality of use of these instruments.

According to the present invention, these objects are achieved by means of an amplifier-transducer device having the characteristics stated in Claim 1, and a detector device, particularly for auscultation, having the characteristics stated in Claim 7.

The hydraulic press amplifier proposed by the invention provides, in a very simple way, amplification with a high factor which permits the construction of instruments which would be impossible if conventional methods were used.

The detector device according to the invention can be used to detect elastic waves (sound waves and other vibrations at inaudible frequencies) and small mechanical oscillations in general.

The detector device for auscultation has an amplification unit which can linearize audibility, since it provides compensation in the form of additional energy at the frequencies for which the human ear has lower detection capacity.

It therefore permits auscultation over a much wider range of frequencies, since both the low tones and the high tones are easily audible. Such a wide range of audible frequencies makes it possible to identify a considerable variety of pathological sounds immediately and in a simple way, by using the discriminatory capacity of the auditory apparatus to the full.

Further characteristics and advantages of the invention will be revealed more fully in the following detailed description, provided by way of non-limitative example, with reference to the attached drawings, in which:
Figure 1 is a diagram showing the curve of loudness,
Figure 2 is a simplified view of an auscultation device according to the invention, showing its principal components;
Figure 3 is an enlarged sectional view of a detail of the device of Figure 2; and
Figures 4 and 5 are two schematic examples of the application of the device of Figure 2.

A detector device for auscultation according to the invention is indicated as a whole by 10, and comprises an auscultation capsule 12 pivoted on a main body 14 provided with a pair of control knobs 16 and 18, for regulating the tone and the volume of the auscultation respectively. A tubular line 20 enables the main body of the device to communicate with a headset 22 provided with a pair of earpieces 24.

The device 10 has an electrical input socket IN and an electrical output socket OUT for connection to external accessory electronic devices, for example a second auscultation capsule 26.

It is also provided with a small screen 30 for displaying information relating to the auscultation signals.

An LED 32 indicates the operating status of the device.

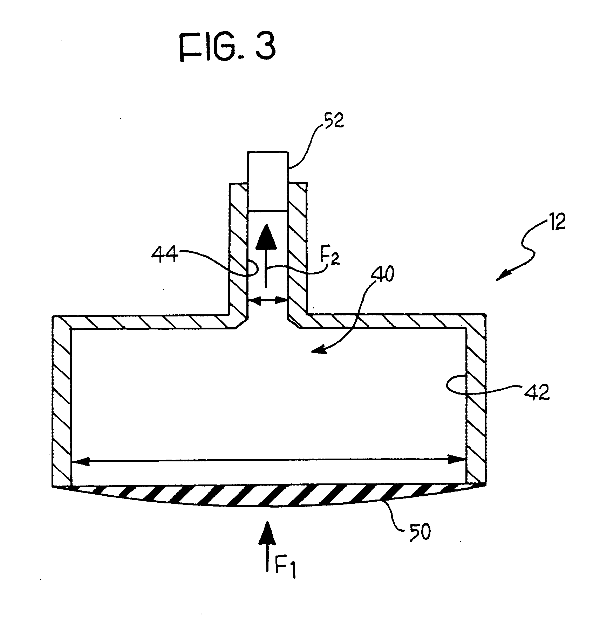

To enable the innovative characteristics of the device to be understood, Figure 3 shows an enlarged sectional schematic view of the auscultation capsule 12.

This capsule comprises a chamber 40 for collecting acoustic signals, this chamber being designed for application to the body or machine to be auscultated, and capable of collecting the acoustic signals generated by the internal organs of this body or machine.

In the following description, the term "acoustic signals" will be used to denote, in a general way, the propagation of elastic waves, regardless of whether or not they reach the human ear and are therefore perceived as sound, and this term is also extended to cover phenomena relating to elastic waves at inaudible frequencies.

The chamber 40 is shaped in such a way as to have a first and a second cylindrical portion, indicated by 42 and 44 respectively, these portions being coaxial and positioned in sequence. The first cylindrical portion 42 has a circular cross section with a diameter greater than the diameter of the circular cross section of the second portion 44. The first portion is closed at its free end by an elastic membrane 50, and at the opposite end it communicates with the second cylindrical portion 44, which, at its end opposite the connection to the first chamber portion 42, houses a microphone 52 for converting the collected acoustic signal into an electrical signal for subsequent processing.

The microphone 52 is a capacitor microphone, and has its moving part exposed to the sound field through the face directed towards the inside of the cylindrical portion 44 of the chamber for collecting the acoustic signal.

The moving part of the microphone is positioned in such a way that, at each point of its travel or oscillation, it occupies the whole cross section of the cylindrical portion 44 in which it is housed, thus forming, together with the membrane 50, a closed acoustic chamber, with no loss or leakage of the fluid contained therein.

In the preferred case of a capacitor microphone, the moving part is one of the two conducting plates of the electrical capacitor which forms its transducer element. The variation of the sound frequencies and pressure due to the intensity of the detected acoustic signal results in a corresponding variation of the capacitance of the capacitor and therefore of the voltage across the said capacitor. Thus the sound frequencies are converted into a modulation of voltage.

An electronic amplification and filter circuit is connected down-line of the microphone for processing the detected signal and filtering by frequency its undesired components according to solutions which are well known in the prior art and which are therefore not described in detail here.

An electro-acoustic converter circuit (not shown) converts the processed electrical signal to an acoustic signal which can be transmitted through the headset 22 to the user's ear.

When the pressure exerted by the fluid contained in the chamber 40 varies as a result of the propagation of the audio frequencies, the moving plate of the capacitor is subjected to a degree of movement proportional to the force acting on it, and consequently the capacitor will have a variable capacitance and therefore a variable voltage across its terminals. For practical purposes, and in this example in any case, the energy required to move the moving plate of the capacitor can be considered to be zero with a good degree of approximation.

An increase in the amplitude of oscillation of the moving part of the capacitor results in an increase in the amplitude of the converted electrical signal.

The particular configuration of the acoustic signal collection chamber is similar to that of a hydraulic press, whose operation is an application of Pascal's principle, namely that, given two cylinders having different cross sections, connected together and each provided with a moving piston, when a force F₁ is applied to the piston with the larger cross section, a force F₂ acts on the piston with the smaller cross section, and this force decreases according to the ratio between the two moving sections of the pistons. Assuming that the volume of fluid displaced is constant, if no appreciable force is required in the system to move the pistons, the excursion of the piston of smaller cross section will be amplified by a factor equal to the ratio between the cross sections of the pistons.

By way of example, we may consider the case of a needle-type measuring instrument (ammeter) used in the prior art for displaying and amplifying the small current excursions of a source under investigation. If the needle is moved without any need for the application of force, due to its low weight and the use of anti-friction bearings, then the visual amplification is obtained by using a series of lever mechanisms positioned in such a way as to divide the original force applied to the needle but to increase its excursion, thus showing a larger movement.

In the operation of the auscultation device, the pistons are replaced by the membrane 50 of the capsule and by the moving part of the microphone respectively. The oscillations of the elastic membrane 50 are transmitted through the fluid contained in the chamber and converted into oscillations of the moving part of the microphone facing the chamber, and are amplified by a quantity equal to the ratio between the larger and smaller cross sections of the two portions of the chamber. Thus the capsule of the device forms a pressure amplifier with a high amplification factor, permitting the construction of an auscultation instrument having high amplification but limited dimensions.

The choices made regarding the cross sections of the chambers 42 and 44, the ratio between the said cross sections, and the volumes of the chambers are determined in a practical way by the elasticity of the membrane 50 and the type of microphone used. A person skilled in the art will understand that these parameters must be chosen in a way which does not take the microphone outside its operating range.

Due to the positioning of the microphone and the consequent complete closure of the chamber 40, there are no pressure losses, and the movements of the membrane 50 are amplified and transferred fully to the moving member of the microphone.

It should be noted that the elastic membrane 50 could be omitted, and replaced by another type of membrane not integrated with the device, but having similar functions in use: for example, in a stethoscope with an open terminal cone, the patient's skin on which the stethoscope rests can correctly act as an elastic membrane, forming a chamber 40 which is closed when the patient's heartbeat or respiration is listened to.

Clearly, the acoustic signal collection chamber can contain air at ordinary ambient pressure, but for special applications, such as sound detection deep underwater, or applications for military use, it can also contain liquids at adjustable pressures.

It will be evident to a person skilled in the art that the principles described above can also be applied to devices including other types of transducers, or simply other types of microphones, such as piezoelectric microphones, and not exclusively to capacitor microphones.

When the detected acoustic signal has been converted to an electrical signal, the volume and tone can be regulated by means of the knobs 16 and 18 for a correct analysis of the signals. These controls can be provided in the form of sealed devices for applications in environments in which there is a high risk of exposure to liquids. The controls can also be provided in the form of digital controls rather than rotary knobs, to facilitate the input of parameters and to enable the settings to be stored.

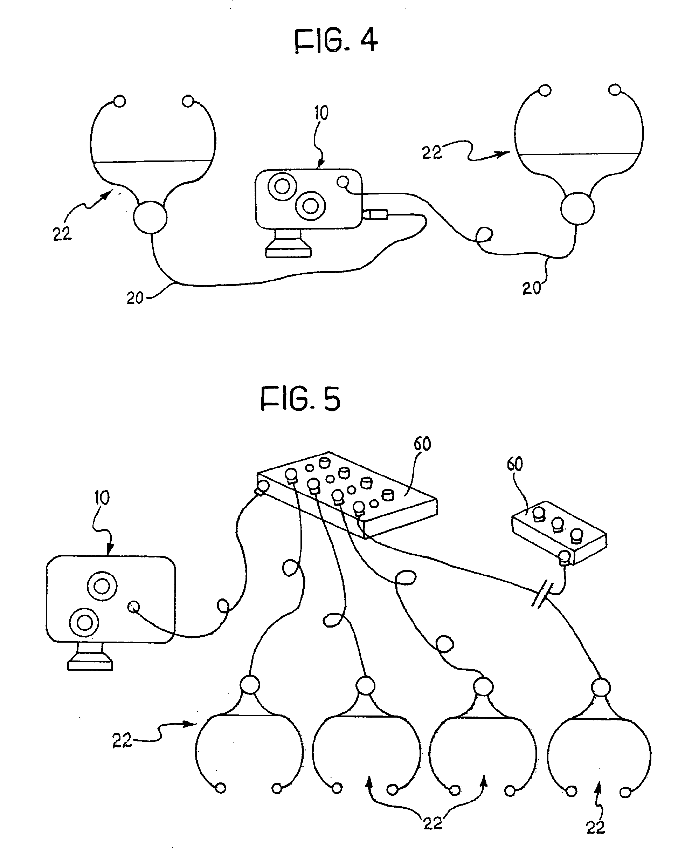

The OUT socket can be used for transmitting electrical signals to peripheral devices, for example for recording on audio media or printing on paper, for display on a monitor, for remote auscultation via a modem, or for simultaneous auscultation by two or more users, by means of a distribution circuit 60 as shown in Figures 4 and 5.

The IN socket enables a capsule 26 similar to the cone of a stethoscope to be used for more localized and precise auscultation without any acoustic deformations which might be introduced by the presence of the membrane incorporated in a phonendoscope.

Advantageously, a pneumatic diverter is provided up-line of the microphone in the cylindrical chamber of smaller cross section 44, for diverting the acoustic signal towards a guide tube in order to prevent the acousto-electric conversion and enable the device to be used in the conventional way without electronic amplification.

The device can be functionally enhanced by the provision of a heartbeat counter and a corresponding display to show the number of heartbeats detected.

The elastic membrane 50 can advantageously be joined to the capsule by means of a threaded fastening ring, allowing it to be removed fairly simply for auscultation without a membrane.

A typical medical application of the detector device proposed by the invention is that of phonendoscopy for the detection and auscultation of heartbeats and respiratory sounds of a living creature.

In general, the device is suitable for use in the fields of cardiology, paediatrics, prenatal paediatrics, pneumology, dental and oral medicine, electromyography, emergency treatment and resuscitation.

The OUT socket can be used for communication with peripheral devices, for example in order to operate the device as a diagnostic tool with facilities for certification, wider consultation, and remote monitoring and data transmission. In particular, this socket can be connected to a local loudspeaker when, for special reasons, it is impossible to wear a headset or preferable not to wear one; or it can be connected to a multiple distributor 60 for distributing the signal to a plurality of headsets or other listening devices. Sound recording is also possible.

A second important application of the detector device according to the invention is that relating to its industrial use as a device for testing machines and mechanical systems in general.

On this subject, we must make some preliminary remarks concerning the fault/noise relationship of a machine and its perception by the human ear.

Any anomaly of a mechanical system introduces a change in the sound produced by the system, in respect of intensity, frequency and rhythm.

The intensity is considered to be a louder noise, the frequency is considered to be the tone, in other words the variation of the sound, and the rhythm is considered to be an added sonority having a different rate from the base sound. The rhythm is the most important element for fault recognition, and the only element which can aid an operator when the sounds produced by the system are not known in advance. This is because, when a technician is familiar with the system on which he has to operate, a simple sound different from the known sounds will be enough to tell him that a fault has occurred. In all other cases, however, it is possible to try to trace faults by means of the detection of the added rhythm. This is because the fault causes the emission of a sound having a rhythm different from the base rhythm in the operation of the mechanical system. In other words, a machine with a fault emits at least two sounds having different cycles.

An oscilloscope, a frequency meter or other analytical instruments can be connected to the OUT socket of the device.

Applications of the detector device proposed by the invention in the industrial field relate essentially to the maintenance of mechanical installations or systems, such as quick-break switches, the opening and closing of contacts and/or valves, electric motors or generators, relays, fluid pumping systems, air conditioning installations, gearing, roller conveyors and all other types of conveyor, automatic machines and robots, private, agricultural and industrial vehicles, oil hydraulic systems, toys, printers, etc.

Clearly, provided that the principle of the invention is retained, the forms of application and the details of construction can be varied widely from what has been described and illustrated purely by way of non-limitative example, and such variation does not constitute a departure from the scope of protection of the present invention as defined by the attached claims.

This is particularly true as regards the possibility of using the device for amplifying mechanical oscillations in general and converting them to electrical signals, by replacing the vibrating membrane positioned in the detecting section of the chamber 40 with an oscillating moving part, such as a piston, for intercepting the mechanical oscillations of a member of a machine.

## Claims

1. Device for amplifying elastic waves or mechanical oscillations in general and converting them into electrical signals, comprising:
a rigid cavity forming a chamber (40) for collecting an elastic wave, or for intercepting a mechanical oscillation, this chamber being suitable for application to a body or a machine to be examined, and being arranged to collect, by means of an oscillating moving member (50) positioned in a detecting section, the elastic waves or mechanical oscillations generated by members of the body or machine, and
transducer means (52) housed inside the said chamber (40), including a moving element for converting the elastic wave or intercepted mechanical oscillation into an electrical and/or electromagnetic signal for subsequent processing,
in which the said chamber (40) is shaped to house the said transducer means (52) in such a way that they are exposed to a field of elastic waves inside the chamber (40) only through the said moving element,
in which the said moving element has a surface exposed to the field of elastic waves which has a smaller area than the area of the detection section of the cavity, the excursion of the said element being increased, with respect to the excursion of the oscillating member (50) positioned in the detection section, according to the ratio between the area of the detection surface and the area of the moving element exposed to the field of elastic waves, and in which
the said chamber (40) is arranged to prevent the dispersion outside the chamber of the elastic waves produced therein.

2. Device according to Claim 1, in which the said chamber (40) is shaped in such a way as to house the said transducer means (52) in a portion of cylindrical chamber (44) having a smaller cross section than the detection section.

3. Device according to Claim 2, in which the said chamber (40) has a first and a second cylindrical portion (42, 44) which are coaxial and have different cross sections, and which are positioned in sequence.

4. Device according to any one of the preceding claims, in which the said transducer means comprise a microphone (52).

5. Device according to Claim 4, in which the said microphone (52) is a capacitor microphone.

6. Device according to any one of the preceding claims, in which the chamber for collecting elastic waves (40) has a removable vibrating elastic membrane (50) as its oscillating moving member.

7. Detector device, in particular an auscultation device, comprising a device for amplifying elastic waves or mechanical oscillations in general and converting them into electrical signals according to any one of Claims 1 to 6.

8. Detector device according to Claim 7, comprising, up-line of the transducer means (52), diverter means for diverting a detected acoustic signal towards a tubular guide line coupled to one or more listening devices, and for excluding the said transducer means (52) from the path of the acoustic signal.

9. Detector device according to Claim 7 or 8, comprising means for processing electrical signals, positioned down-line of the said transducer means (52) and including an amplification and filter circuit for further amplification of the signal and elimination of the undesired frequency components.

10. Detector device according to any one of Claims 7 to 9, comprising an electrical signal output terminal (OUT) for connection to peripheral devices for signal processing, remote transmission or display.
